# FASCICULE DE BREVET EUROPEEN

(11) **EP 1 093 775 B1**
(45) Date de publication et mention de la délivrance du brevet: **22.02.2006**
(21) Numéro de dépôt: 00402890.8
(22) Date de dépôt: 19.10.2000
(51) Int. Cl.: A61F 2/16

(54) **Implant intraoculaire**
Intraokulares Implantat
Intraocular Implant

(30) Priorité: 21.10.1999 FR 9913125
(43) Date de publication de la demande: 25.04.2001
(73) Titulaire: Humanoptics AG, 61054 Erlangen (DE)
(72) Inventeur: Hanna, Khalil, 75007 Paris (FR)
(74) Mandataire: Rau, Albrecht

(56) Documents cités:
- WO-A-97/26842
- FR-A- 2 728 458
- FR-A- 2 776 181
- FR-A- 2 778 093
- FR-A- 2 784 575
- GB-A- 2 215 076
- US-A- 4 878 912

## Description

La présente invention concerne un implant oculaire formant cristallin artificiel pour être logé dans le sac capsulaire après opération de la cataracte.

Parmi les nombreuses techniques existant pour effectuer l'opération de la cataracte, l'une d'elles consiste à extraire la matière cristallinienne du sac capsulaire après avoir réalisé une ouverture circulaire (capsulorhexis) dans la paroi antérieure de ce sac. La partie préservée du sac capsulaire se compose donc de la paroi postérieure et d'une couronne de paroi antérieure reliée à la paroi postérieure par la paroi équatoriale du sac qui coopère avec le muscle ciliaire par les fibres zonulaires.

Après l'extraction de cette matière cristallinienne, on met généralement en place dans ce qui reste du sac capsulaire une lentille artificielle (un implant) comportant une partie optique centrale équipée de parties dites haptiques qui servent à maintenir l'optique dans le sac capsulaire en prenant appui sur la face interne de la zone équatoriale du sac capsulaire.

Ces parties haptiques peuvent prendre de nombreuses formes. Parmi les plus courantes, on citera des anses élastiques qui partent radialement de la partie optique en se recourbant pour présenter une partie relativement longue venant s'appuyer élastiquement sur le diamètre interne du sac capsulaire. D'autres sont formées par des bras radiaux régulièrement répartis autour de la partie optique qui se terminent par un bourrelet externe en forme d'arc circulaire prenant appui sur la zone équatoriale du sac. Le document FR 2776181 décrit un implant intraoculaire correspondant au préambule de la revendication 1.

On sait que la présence de cellules épithéliales qui tapissent la surface interne du sac capsulaire au niveau de la zone équatoriale donnent naissance à une croissance de cellules à l'intérieur du sac, croissance appelée fibrose. Cette fibrose est très différente d'un sujet à l'autre et son importance peut être estimée par le chirurgien au cours d'un examen clinique de son patient. Elle est en général plus importante dans la zone équatoriale du sac où les cellules germinatives sont logées.

L'une des conséquences de cette fibrose réside dans la luxation ou l'expulsion progressive de l'implant hors du sac capsulaire. L'une des qualités que doit présenter un implant réside donc dans sa capacité à prendre en compte cette fibrose soit pour la limiter soit pour s'en servir afin de mieux assujettir l'implant dans le sac capsulaire.

L'implant selon l'invention offre ces qualités et, en outre, par sa structure, permet au chirurgien en fonction du diagnostic qu'il a réalisé, d'adapter l'aptitude de l'implant à s'accommoder de l'importance estimée plus ou moins grande de la fibrose.

A cet effet l'invention a donc pour objet un implant intraoculaire comportant en une seule pièce, une partie centrale optique et au moins deux parties haptiques, dans lequel l'extrémité libre de chaque partie haptique est située sur un cercle caractérisé en ce que l'extrémité libre de chaque partie haptique est conformée en gouttière, s'étendant le long d'un cercle centré sur l'axe de la partie optique.

L'une des fonctions de cette gouttière est de maintenir séparées l'une de l'autre, au voisinage de la zone équatoriale du sac capsulaire, l'anneau antérieur subsistant après capsulorhexis et la paroi postérieure. Cette forme en gouttière permet de préserver à l'implant, dans ses parties, une souplesse suffisamment grande pour permettre son pliage aisé facilitant son introduction dans l'oeil, ce qu'à dimensions égales un bourrelet ne permettrait pas. Un autre avantage de cette gouttière réside dans la possibilité de loger une pièce secondaire dans l'implant en forme d'anneau dans le cas où le chirurgien estime grande la probabilité d'une fibrose importante. Cet anneau permet de limiter la croissance des cellules et de renforcer les extrémités des parties haptiques, augmentant ainsi leur capacité à résister à la poussée fibrotique. Un anneau plein mis en place dans les gouttières de chaque partie haptique permet également de diminuer les épaisseurs de ces parties haptiques.

D'autres caractéristiques et avantages de l'invention ressortiront à la lecture de la description donnée ci-après de deux exemples de réalisation de l'invention.

Il sera fait référence aux dessins annexés parmi lesquels :
- les figures 1 et 2 sont deux vues extérieures opposées d'un premier mode de réalisation de l'invention,
- les figures 3 et 4 illustrent la mise en place d'un anneau dans l'implant représenté aux figures 1 et 2,
- les figures 5 et 6 sont deux vues opposées extérieures d'une seconde variante de réalisation de l'implant selon l'invention et,
- les figures 7 et 8 illustrent cette deuxième variante d'implant équipée d'un anneau de renforcement.

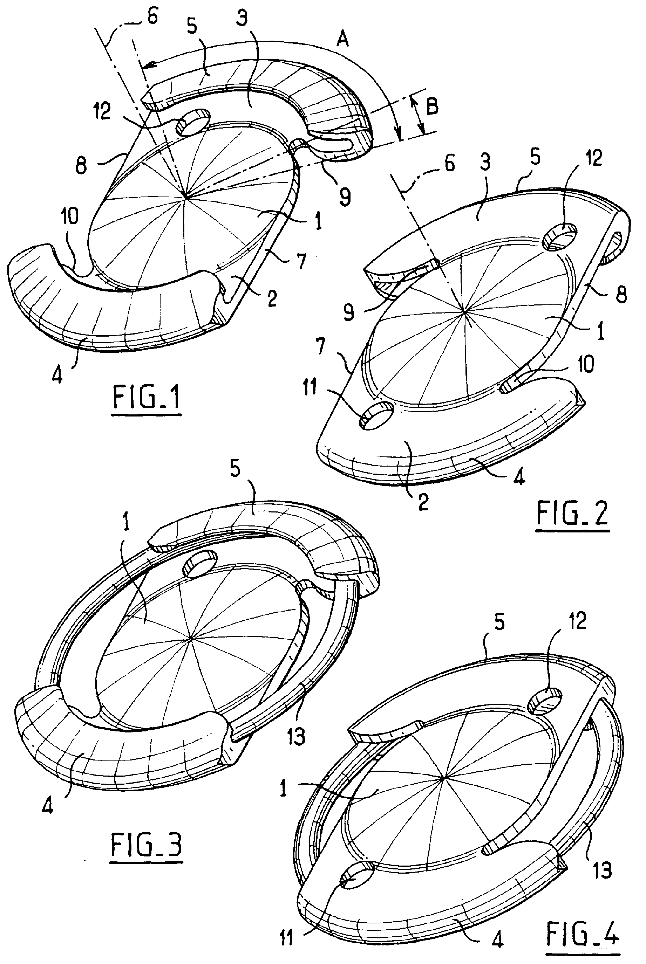

L'implant représenté aux figures 1 à 4 comporte en une seule pièce, une partie optique centrale 1 en forme générale par exemple d'une lentille biconvexe de diamètre de l'ordre de 6 mm, équipée de deux parties haptiques 2 et 3 diamétralement opposées terminées par un bord 4, 5 ourlé ou en forme de gouttière curviligne, s'étendant le long d'un cercle centré sur l'axe 6 de la partie optique. A titre d'exemple, le diamètre extérieur dans lequel s'inscrit l'implant est de l'ordre de 10 mm à 10,5 mm alors que les fonds de chaque gouttière s'étendent le long d'un cercle concentrique au cercle extérieur d'un diamètre de l'ordre de 9 à 9,5mm. Chaque partie haptique est symétrique de l'autre par rapport à l'axe 6 et la longueur angulaire A de chaque gouttière est de l'ordre de 100°. La portion qui relie chaque gouttière à la partie optique s'étend dans le plan médian de la lentille optique 1 et est limitée latéralement par un bord 7, 8 tangent à la partie optique et par un autre bord 9, 10 qui est encoché, la profondeur angulaire de l'encoche B étant de l'ordre de 20°. Cette portion de partie haptique intermédiaire entre la partie optique et la gouttière, possède une ouverture 11, 12 qui, avec les encoches susdites, permet de faire tourner l'implant à l'intérieur du sac capsulaire.

L'épaisseur de l'implant prise au niveau des gouttières est à titre d'exemple de l'ordre de 1,5 mm. L'épaisseur des parties haptiques mesurées dans leur portion intermédiaire est de l'ordre de 0,3 mm.

L'implant selon l'invention et en tant que de besoin, peut comprendre également un anneau 13 de forme générale toroïdale en matière élastiquement déformable dont le diamètre est adapté au diamètre en fond de gouttière de l'implant et dont la section est sensiblement circulaire de diamètre correspondant à la largeur des gouttières susdites.

L'implant est réalisé dans une matière connue en elle-même possédant les qualités optiques de réfraction nécessaires à former une lentille possédant des caractéristiques définies, et des qualités de souplesse permettant le pliage de l'implant et de l'anneau de manière qu'il puisse ne présenter qu'une faible dimension pour son insertion au travers d'une incision de l'oeil relativement petite.

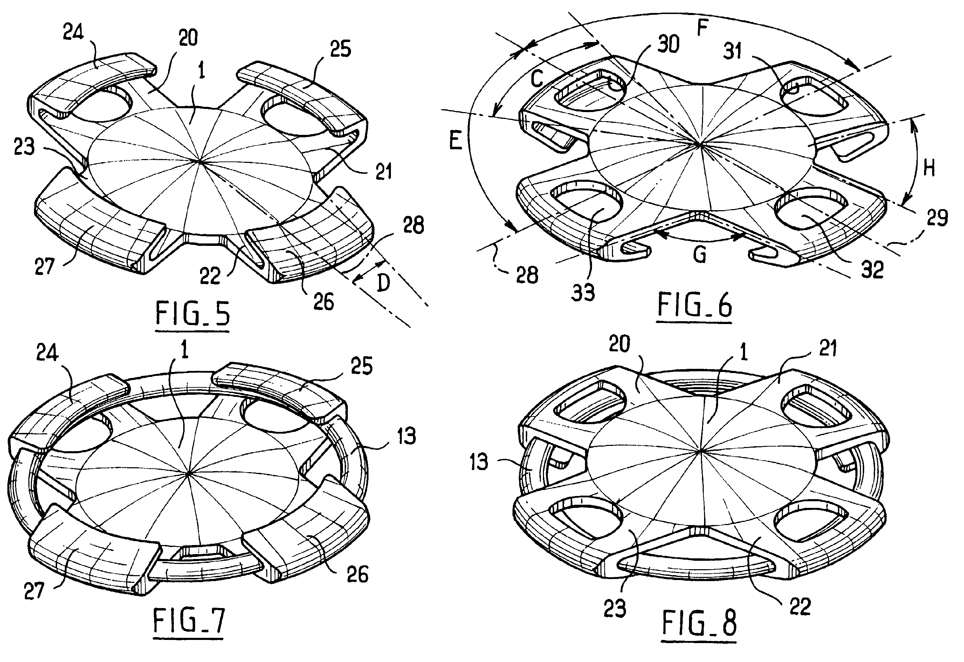

La variante de réalisation de l'implant selon l'invention représentée aux figures 5 à 8 est semblable à celle déjà décrite en ce qu'elle comporte une partie optique centrale 1 en forme de lentille biconvexe par exemple et des parties haptiques 20, 21, 22, 23 également ourlées à leur extrémité pour former des gouttières 24, 25, 26, 27 tout à fait semblables à celles 4 et 5 décrites précédemment. La longueur circonférentielle angulaire de chaque partie haptique notée C aux figures est de l'ordre de 40° dans le mode de réalisation représenté, et la partie de liaison de chaque gouttière à la partie optique centrale est limitée par deux bords latéraux parallèles sensiblement radiaux à l'inclinaison près d'un angle D de l'ordre de 7° à 8° par rapport au rayon médiateur 28 de chaque gouttière.

On notera que les quatre bras haptiques de cette variante de réalisation ne sont pas régulièrement répartis autour de la partie optique mais forment un X. Deux bras haptiques opposés ont le même diamètre médiateur 28 et 29 et l'angle au centre formé entre ces deux diamètres est de l'ordre de 80° (l'angle E) ou son supplémentaire c'est-à-dire 100° (l'angle F). Ainsi, les bords de deux parties haptiques voisines forment entre eux en angle G de l'ordre de 115° ou H de l'ordre de 65°.

De même que pour le mode de réalisation précédent, chaque bras haptique possède une ouverture 30, 31, 32, 33.

L'anneau 13 décrit en regard des figures précédentes peut également être adjoint à ce type d'implant comme l'illustrent les figures 7 et 8. Il y joue le même rôle.

Enfin, l'implant selon l'invention est destiné à être implanté de manière que les ailes ou lèvres libres de chaque gouttière soient situées en regard de la paroi antérieure du sac capsulaire, c'est-à-dire que la face de l'implant visible aux figures 2, 4, 6 et 8 est tournée du côté de la paroi postérieure de la capsule.

## Revendications

1. Implant intraoculaire comportant en une seule pièce, une partie centrale optique (1) et au moins deux parties haptiques (2, 3), l'extrémité libre de chaque partie haptique (2, 3) est située sur un cercle **caractérisé en ce que** l'extrémité libre de chaque partie haptique est conformée en gouttière (4,5), s'étendant le long d'un cercle centré sur l'axe (6) de la partie optique.

2. Implant intraoculaire selon la revendication 1, **caractérisé en ce que** chaque partie haptique (2, 3) est pourvue d'un orifice (11, 12) entre la gouttière (4, 5) et la partie optique (1).

3. Implant selon la revendication 1 ou la revendication 2, **caractérisé en ce qu'**il comporte deux parties haptiques symétriques l'une de l'autre par rapport à l'axe central (6) de la partie optique (1), chacune avec une gouttière (4, 5) de longueur angulaire A d'environ 100°, un bord latéral (8) tangent à la partie optique (1) et un autre bord latéral (9) encoché sur environ un angle B de 20°.

4. Implant selon la revendication 1 ou la revendication 2, **caractérisé en ce qu'**il comporte quatre parties haptiques (20, 21, 22, 23) réparties autour de la partie optique (1), de longueur circonférentielle angulaire C de l'ordre de 40° et limitées par deux bords latéraux parallèles inclinés par rapport au rayon médiateur (28) de leur gouttière d'un angle D d'environ 7° à 8°.

5. Implant intraoculaire selon la revendication 4, **caractérisé en ce que** les rayons médiateurs de deux parties haptiques opposées sont sur un même diamètre (28, 29) de l'implant, ces deux diamètres se coupant sous un angle d'environ 100° (ou 80°) et **en ce que** les bords de deux parties haptiques voisines forment entre eux un angle de l'ordre de 115° (ou 65°).

6. Implant intraoculaire selon l'une des revendications précédentes, **caractérisé en ce qu'**il comporte un anneau (13) élastiquement déformable, indépendant, susceptible d'être logé dans la gouttière de chacune des parties haptiques.

## Claims

1. An intraocular implant, in one piece comprising a central optic (1) and at least two haptics (2, 3), the free end of each of the haptics (2, 3) being located on a circle, **characterized in that** the free end of each of the haptics forms a groove (4, 5) which runs on a circle that is disposed around the axis (6) of the optic.

2. An intraocular implant according to claim 1, **characterized in that** each haptic (2, 3) is provided with an orifice (11, 12) between the groove (4, 5) and the optic (1).

3. An implant according to claim 1 or claim 2, **characterized in that** it comprises two haptics which, in relation to the central axis (6) of the optic (1), are symmetrical one to the other, each having a groove (4, 5) of an angular area A of approximately 100°, a lateral edge (8) tangent to the optic (1) and another lateral edge (9) which is bent approximately by an angle B of 20°.

4. An implant according to claim 1 or claim 2, **characterized in that** it comprises four haptics (20, 21, 22, 23) of a peripheral angular area C in the range of 40° which are distributed around the optic (1) and defined by two parallel lateral edges which incline in relation to the mean radius (28) of their groove by an angle D of approximately 7° to 8°.

5. An intraocular implant according to claim 4, **characterized in that** the mean radiuses of two opposed haptics are on a same diameter (28, 29) of the implant, with these two diameters intersecting by an angle of approximately 100° (or 80°); and **in that** the edges of two adjacent haptics make an angle in the range of 115° (or 65°).

6. An intraocular implant according to one of the preceding claims, **characterized in that** it comprises an independent, elastically deformable ring (13) that is able to be placed in the groove of each of the haptics.

## Patentansprüche

1. Intraokulares Implantat, das einstückig eine zentrale Optik (1) und wenigstens zwei Haptiken (2, 3) umfasst, wobei das freie Ende jeder Haptik (2, 3) auf einem Kreis liegt, **dadurch gekennzeichnet, dass** das freie Ende jeder Haptik eine Nut (4, 5) bildet, die sich über einen um die Achse (6) der Optik angeordneten Kreis erstreckt.

2. Intraokulares Implantat nach Anspruch 1, **dadurch gekennzeichnet, dass** jede Haptik (2, 3) mit einer Öffnung (11, 12) zwischen der Nut (4, 5) und der Optik (1) versehen ist.

3. Implantat nach Anspruch 1 oder Anspruch 2, **dadurch gekennzeichnet, dass** es zwei, zur Mittelachse (6) der Optik (1) zueinander symmetrische Haptiken umfasst, wobei jede mit einer sich über einen Winkelbereich A von etwa 100° erstreckenden Nut (4, 5), einem die Optik (1) tangierenden Seitenrand (8) und einem weiteren, etwa über einen Winkel B von 20° umgebogenen Seitenrand (9) versehen ist.

4. Implantat nach Anspruch 1 oder Anspruch 2, **dadurch gekennzeichnet, dass** es vier um die Optik (1) verteilte Haptiken (20, 21, 22, 23) mit einem Umfangswinkelbereich C von 40° umfasst, die von zwei parallelen Seitenrändern begrenzt sind, die zum Mittelhalbmesser (28) ihrer jeweiligen Nut um einen Winkel D von etwa 7° bis 8° geneigt sind.

5. Intraokulares Implantat nach Anspruch 4, **dadurch gekennzeichnet, dass** die Mittelhalbmesser von zwei jeweils einander gegenüberliegenden Haptiken auf einem gleichen Durchmesser (28, 29) des Implantats liegen, wobei diese beiden Durchmesser sich mit einem Winkel von etwa 100° (oder 80°) schneiden, und dass die Ränder von zwei benachbarten Haptiken zwischen sich einen Winkel von 115° (oder 65°) bilden.

6. Intraokulares Implantat nach einem der vorhergehenden Ansprüche, **dadurch gekennzeichnet, dass** es einen eigenständigen, elastisch verformbaren Ring (13) aufweist, der in der Nut jeder Haptik aufnehmbar ist.
